# EUROPEAN PATENT APPLICATION

(11) **EP 3 673 983 A1**
(43) Date of publication of application: **01.07.2020**
(21) Application number: 18847749.1
(22) Date of filing: 13.08.2018
(51) Int. Cl.: B01F 5/20, B01F 3/04, C01B 3/10, A61L 9/14

(54) **DEVICE AND METHOD FOR MANUFACTURING HIGH-PRESSURE ATOMIZED OZONE WATER**

(30) Priority: 22.08.2017 CN 201710728511
(71) Applicant: GRN Consultant (Hong Kong) Limited, Fanling, Hong Kong (CN)
(72) Inventor: CHAN, Chi Kwong, Fanling Hong Kong (CN); WONG, Alan Yuk Chun, Fanling Hong Kong (CN)
(74) Representative: Herrmann, Jochen
(86) International application number: PCT/CN2018/100251
(87) International publication number: WO 2019/037614

(57) **Abstract**

The invention provides a device and a method for producing atomized ozonated water by high pressure and relates to the field of environmental management. The device comprises a first pressurizing device, an ozone generator, a water supply device, a first pipeline, a second pipeline, and a spraying device. The first pressurizing device is connected with the ozone generator. The first pipeline is connected with the ozone generator and the spraying device. The second pipeline is connected with the water supply device and the spraying device. The first pressurizing device is used to feed compressed air into the ozone generator. The ozone generator is used to convert part of the compressed air into compressed ozone, and feed the compressed ozone or unconverted compressed air into the spraying device. The water supply device is used to feed water or ozonated water into the spraying device such that either the water is mixed with the compressed ozone in the spraying device to form atomized ozonated water, or the ozonated water is mixed with the unconverted compressed air in the spraying device to form atomized ozonated water. The present invention can provide a cleansing element for air purification, dissolution of chemical gases and assurance of environmental hygiene.

## Description

### Field

The present invention relates to the field of environmental management, and in particular to a device and a method for producing atomized ozonated water by high pressure.

### Background technology

With the development of society, environmental pollution is becoming more and more serious, and odor is one of the environmental problems that directly affects public health.

Currently, environmental disinfection is mostly carried out by using chemicals in a sterilization device or spraying disinfectant by vehicles. However, these methods are not only costly but also produce by-products after disinfection, causing secondary harmful effects to the environment and human.

### The present invention

The present invention aims to provide a device and a method for producing atomized ozonated water by high pressure, which provide a cleansing element for air purification, dissolution of chemical gases and assurance of environmental hygiene.

Embodiments of the present invention are implemented as follows:
Firstly, an embodiment of the present invention provides a device for producing atomized ozonated water by high pressure, which comprises a first pressurizing device, an ozone generator, a water supply device, a first pipeline, a second pipeline, and a spraying device. The said first pressurizing device is connected with the said ozone generator. The inlet of the said first pipeline is connected with the said ozone generator. The outlet of the said first pipeline is connected with the said spraying device. The inlet of the said second pipeline is connected with the said water supply device. The outlet of the said second pipeline is connected with the spraying device. The said first pressurizing device is used to feed compressed air into the said ozone generator. The said ozone generator is used to receive the said compressed air, converting part of the compressed air into compressed ozone, and feeding the said compressed ozone or unconverted compressed air into the said spraying device through the first pipeline. The water supply device is used to feed water or ozonated water into the said spraying device through the said second pipeline such that either the said water is mixed with the said compressed ozone in the spraying device to form atomized ozonated water, or the said ozonated water is mixed with the said unconverted compressed air in the spraying device to form atomized ozonated water. In a preferred embodiment of the present invention, the said device for producing atomized ozonated water by high pressure also comprises a first valve and a third pipeline. The inlet of the said third pipeline is connected with the said first pressurizing device. The outlet of the said third pipeline is connected with the said ozone generator. The said first valve is configured in the said third pipeline. The said first valve is used to adjust the pressure of the said first pressurizing device on the ozone or the unconverted air in the said ozone generator in order to control the concentration of atomized ozonated water formed at the said spraying device.

In a preferred embodiment of the present invention, the said device for producing atomized ozonated water by high pressure also comprises a second valve. The said second valve is configured in said second pipeline. The said second valve is used to adjust the flow rate of the said water or the said ozonated water from the said water supply device in order to control the concentration of the atomized ozonated water formed at the said spraying device.

In a preferred embodiment of the present invention, the said device comprises a second pressurizing device. The said second pressurizing device is connected with the said water supply device. The said second pressurizing device is used to pressurize the said water supply device to control the flow rate of the said water or the said ozonated water from the water supply device.

In a preferred embodiment of the present invention, the said device for producing atomized ozonated water by high pressure also comprises an ultraviolet lamp. The said ultraviolet lamp is configured on the outside of the said spraying device to produce ultraviolet light, which reacts with the said atomized ozonated water to produce hydroxyl radicals for decomposition of toxic substances.

In a preferred embodiment of the present invention, the said device for producing atomized ozonated water by high pressure also comprises a cooling device. The cooling device is configured inside the said water supply device. The cooling device is used to cool down the said water or the said ozonated water.

In a preferred embodiment of the present invention, the said device for producing atomized ozonated water by high pressure also comprises an oxygen generation device. The said generation device is configured between and connected with the said first pressurizing device and the said ozone generator. The said oxygen generation device is used to receive compressed air from the said first pressurizing device, and then feeding the oxygen generated from the said compressed air into the said ozone generator.

In a preferred embodiment of the present invention, the said first pressurizing device is an air compressor.

In a preferred embodiment of the present invention, the said spraying device is a fix-mounted spraying device or a mobile pipes-spraying device.
Secondly, an embodiment of the present invention provides a method for producing atomized ozonated water by high pressure, which is applied in a device for producing atomized ozonated water by high pressure. The said device for producing atomized ozonated water by high pressure comprises a first pressurizing device, an ozone generator, a water supply device, a first pipeline, a second pipeline, and a spraying device. The said first pressurizing device is connected with the said ozone generator. The inlet of the said first pipeline is connected with the said ozone generator. The outlet of the said first pipeline is connected with the said spraying device. The inlet of the said second pipeline is connected with the said water supply device. The outlet of the said second pipeline is connected with the spraying device. The said method comprises:
The said first pressurizing device feeding compressed air into the said ozone generator; the said ozone generator receiving the said compressed air, converting part of the compressed air into compressed ozone, and feeding the said compressed ozone or unconverted compressed air into the said spraying device through the first pipeline; the water supply device feeding water or ozonated water into the said spraying device through the said second pipeline such that either the said water is mixed with the said compressed ozone in the spraying device to form atomized ozonated water, or the said ozonated water is mixed with the said unconverted compressed air in the spraying device to form atomized ozonated water.

An embodiment of the present invention provides a device and a method for producing atomized ozonated water by high pressure, which comprises a first pressurizing device, an ozone generator, a water supply device, a first pipeline, a second pipeline, and a spraying device. The first pressurizing device is connected with the ozone generator. The inlet of the first pipeline is connected with the ozone generator. The outlet of the first pipeline is connected with the spraying device. The inlet of the second pipeline is connected with the water supply. The outlet of the second pipeline is connected with the spraying device. By using the first pressurizing device to feed compressed air into the ozone generator, using the ozone generator to receive compressed air and convert part of the compressed air into compressed ozone and feed the compressed ozone or unconverted compressed air into the spraying device through the first pipeline, and using the water supply device to feed water or ozonated water into the spraying device through the second pipeline such that either the water is mixed with the compressed ozone in the spraying device to form atomized ozonated water, or the ozonated water is mixed with the unconverted compressed air in the spraying device to form atomized ozonated water, a cleansing element is provided for air purification, dissolution of chemical gases and assurance of environmental hygiene.

Other features and advantages of the present invention will be explained in the following description. Some will become obvious from the description or could be understood by implementing the present invention. The objectives and other advantages of the present invention can be achieved and obtained through the structure specifically set out in the specification, claims, and drawings.

### Description of Drawings

In order to more clearly illustrate the technical solutions in the embodiments of the present invention, a brief description of the drawings required for the embodiments is set out below. It should be understood that the drawings illustrate only certain embodiments of the present invention. Therefore, they should not be regarded as limiting the scope of the present invention. Ordinary persons skilled in the art may, without input of creative efforts, obtain other drawings according to these drawings. Through the drawings, the said objectives of the present invention and other objectives, features and advantages will become clearer. In all of the drawings, the same reference marks indicate the same parts. The drawings are not drawn to scale; they are only intended to illustrate the crux of the present invention.
FIG. 1 is the first block diagram of the device for producing atomized ozonated water by high pressure provided by the first embodiment of the present invention.
FIG. 2 is the second block diagram of the device for producing atomized ozonated water by high pressure provided by the first embodiment of the present invention.
FIG. 3 is the third block diagram of the device for producing atomized ozonated water by high pressure provided by of the first embodiment of the present invention.
FIG. 4 is the flow chart of the method for producing atomized ozonated water by high pressure provided by the second embodiment of this invention.

### Legend:

100 - a device for producing atomized ozonated water by high pressure; 102 - first pressurizing device; 104 - ozone generator; 106 - water supply device; 108 - first pipeline; 110 - second pipeline; 112 - spraying device; 114 - first valve; 116 - third pipeline; 118 - second valve; 120 - second pressurizing device; 122 - oxygen generation device.

### Detailed Implementation

To illustrate the objectives, technical solutions, and advantages of the embodiments of the present invention more clearly, a clear and complete description of the technical solutions in the embodiments of the present invention is set out below, accompanied by drawings of the embodiments of the present invention. Obviously, the embodiments described below are only a part but not all embodiments of the present invention. Components of embodiments of the present invention described and shown in these drawings can generally be arranged and designed by different configurations.

Accordingly, the detailed description of the embodiments of the present invention below is not intended to limit the scope of protection claimed for the present invention, but merely illustrates the selected embodiments of the present invention. Based on the embodiments of the present invention, all other embodiments obtained by ordinary persons skilled in the art without input of creative efforts also fall within the scope of protection for the present invention.

It should be noted that like reference numbers and letters refer to the like items in the following drawings. Therefore, once an item is defined in one drawing, it does not need to be further defined and explained in subsequent drawings.

In the description of the present invention, it should be noted that terms indicating orientation or position relationship, such as "center", "on", "below", "left", "right", "vertical", "horizontal", "inside", "outside", etc., are based on the orientation or position relationship shown in the drawings, or the habitual orientation or position relationship in using a product of the present invention. Such terms are only used for convenience in describing the present invention and simplifying the description instead of indicating or implying that the device or component must be set in a particular orientation or constructed and operated in a particular orientation, and thus shall not be construed as a limitation on the present invention. In addition, the terms "first", "second", "third", etc. are merely used to make a distinction and shall not be construed as indicating or implying the relative importance.

In addition, the terms "horizontal", "vertical", "overhang" and the like do not mean that components should be absolutely horizontal or vertical; they can be tilted slightly. For example, "horizontal" simply means its direction is more horizontal than "vertical". It does not mean that the structure must be completely horizontal; it can be tilted slightly.

In the description of the present invention, it should also be noted that the terms "configured", "installed", and "connected", etc., shall be broadly construed. For example, it can be a fixed connection, a detachable connection or an integrated connection. It can also be a mechanical connection or electrical connection. It can be connected either directly or indirectly through an intermediate medium, or internally connected between two components. Ordinary persons skilled in the art can understand the specific meaning of the above terms in the present invention according to the particular circumstances.

After long-term research and extensive practice, the inventors found that water mist has the functions of removing dust, lowering temperature, increasing humidity, and dissolving chemical gases, etc. It can be used for air cleansing and purification. Ozone is a naturally-occurring gas. In addition to the ozone layer, which filters ultraviolet light from the sun and protects life on Earth, ozone generated after storms can also refresh the air. Ozone is a refreshing and cleansing element in nature. It can be widely used in disinfection, deodorization, sterilization, decomposition of VOC and toxic gases. However, there are many limitations in the application of ozone gas. For example, the concentration of ozone should not exceed 0.05 ppm in any space with human activities. Ozonated water is produced by dissolving ozone into water by scientific technology to increase the ORP value in water. Generally, the ORP value can reach 650-700m Volt when ozone is dissolved in water. It can be used for drinking water disinfection. When the ORP value of ozonated water reaches 970-1000m Volt, it can be used for industrial laundry.

Therefore, atomization and ozonated water is a perfect combination as water droplets and water mist can be used for air cleansing, dust adhesion, and chemical gases dissolution. It has a comprehensive cleansing ability, and ozone has strong disinfection, deodorization and oxidation power. It can decompose most toxic gases. When water combines with ozone to form ozonated water and further atomized into atomized ozonated water, it can be widely used for air purification, environmental protection, hygiene improvement, agriculture, animal husbandry and so on.

### First embodiment

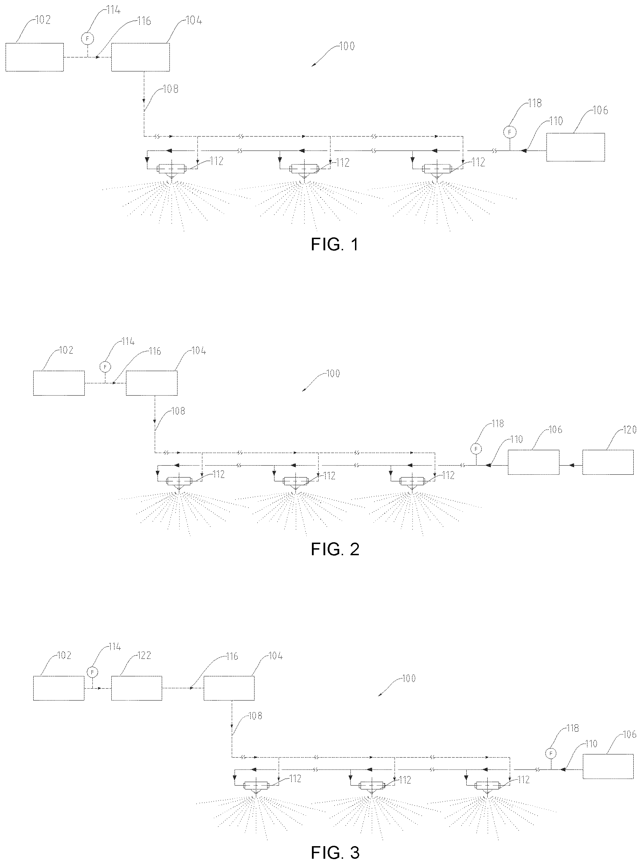

Please refer to FIG. 1. The embodiment provides a device for producing atomized ozonated water by high pressure (100), which comprises a first pressurizing device (102), an ozone generator (104), a water supply device (106), a first pipeline (108), a second pipeline (110) and a spraying device (112).

As an implementation of the present embodiment, the first pressurizing device (102) is connected with the ozone generator (104), the inlet of the first pipeline (108) is connected with the ozone generator (104), the outlet of the first pipeline (108) is connected with the spraying device (112), the inlet of the second pipeline (110) is connected with the water supply device (106), and the outlet of the second pipeline (110) is connected with the spraying device (112). Understandably, the diameter of the first pipeline (108) can be determined depending on the amount of ozone to be transported, thus it is not specified herein. The diameter of the second pipeline (110) can be determined depending on the intended flow rate of the water supply device (106), thus it is not specified herein.

In the present embodiment, the spraying device (112) is connected with the outlet of the first pipeline (108) and the outlet of the second pipeline (110). That means the outlet of the first pipeline (108) and the outlet of the second pipeline (110) are joined at the spraying system (112).

As an implementation of the present embodiment, the first pressurizing device (102) is connected with the ozone generator (104) to feed compressed air into the ozone generator (104). Preferably, the first pressurizing device (102) is an air compressor, which pressurizes the air before it is fed into and processed by the ozone generator (104).

Furthermore, the ozone generator (104) is used to receive compressed air from the first pressurizing device (102) and convert part of the compressed air into compressed ozone. Understandably, air contains a large amount of impurities such as nitrogen and helium. Therefore, when the ozone generator (104) receives compressed air and converts it into compressed ozone, a large amount of impurities will remain unconverted as compressed air. As such, the ozone generator (104) will feed the compressed ozone or the unconverted compressed air into the spraying device (112) through the first pipeline (108).

In the present embodiment, water or ozonated water is stored in the water supply device (106) in advance. In particular, ozonated water can be produced by electrolysis of ozone. In that case, the water supply device (106) is connected with the electrolytic ozone generator, injects water into the electrolytic ozone generator to produce ozonated water, and then feeds the ozonated water into the water supply device (106) for storage. Ozonated water can also be produced by corona discharge of ozone and the use of an ejector. In that case, water is injected into an ejector or an ejection pump and connected with a corona discharge ozone generator to produce high concentration ozone. After ozone is mixed in the ejector or ejection pump, ozonated water is produced. The ozonated water is then fed into the water supply device (106) for storage.

Specifically, the water supply device (106) is used to feed the pre-stored water or ozonated water into the spraying device (112) through the second pipeline (110). Understandably, as the outlet of the first pipeline (108) and the outlet of the second pipeline (110) are joined at the spraying device (112), that means the compressed ozone or the unconverted compressed air from the first pipeline (108) and the water or ozonated water from the second pipeline (110) are joined at the spraying device (112). As a preferred method of the present embodiment, the water from the water supply device (106) and the compressed ozone from the ozone generator (104) are joined at the spraying device (112) to form atomized ozonated water. As another preferred method of the present embodiment, the ozonated water from the water supply device (106) and the unconverted compressed air from the ozone generator (104) are joined at the spraying device (112) to form atomized ozonated water.

Furthermore, the spraying device (112) is used to spray the atomized ozonated water mixed and formed at the spraying device (112) for environmental purification. Understandably, atomized ozonated water can be applied in different types of air purifiers for indoor HVAC deodorization and disinfection, dust and particles removal, and treatment of toxic industrial gases and particles. Atomized ozonated water can also be applied in sprinkler systems. In particular, when atomized ozonated water is used in a sprinkler system, firstly, as the spraying device (112) can directly spray the atomized ozonated water over a large area, it can achieve the purposes of environmental protection and improvement of hygiene. Secondly, as the spraying device (112) can use the atomized ozonated water in farm and greenhouse irrigation and increase humidity, understandably, when atomized ozonated water is used in animal husbandry and food warehouse, it can achieve effective control of environmental hygiene. Usually, when humidity in greenhouse increases, the amount of fungi, bacteria, and even virus also increases. However, there is no such problem with atomized ozonated water, and it can even be used to improve environmental hygiene, sterilization and deodorization, and reducing epidemic and diseases in plants and animals. When the concentration of ozonated water is increased to over 4ppm, it can be used as pesticide, which helps to reduce dependence on pesticides and fungicides and increase the product yield.

Understandably, by adjusting the aperture of the spraying device (112), the diameter of the water droplets of the atomized ozonated water can be adjusted. A smaller aperture of the spraying device (112) will result in highly atomization of fine water droplets for air purification; a bigger aperture of the spraying device (112) will produce bigger water droplets for spraying and cleaning. Therefore, the size of the aperture of the spraying device 112 is not specified but can be changed according to specific needs.

Furthermore, the spraying device (112) can be a fix-mounted spraying device or a mobile pipes-spraying device. In particular, a pipes-spraying device or a mobile pipes-spraying device creates an atomized ozonated water shield for large area environmental and sanitation management, such as sewage treatment plant and landfill deodorization and sterilization. When the spraying device (112) is a fix-mounted spraying device, the spraying device (112) can be directly installed in a designated area, such as landfill, sewage treatment plant, incinerator, and various polluting industries and so on for odor and air pollution control. When the spraying device (112) is a mobile pipes-spraying device, a pointer type and a translation type mobile piles-spraying device are included to operate with a mechanical system to perform a back-and-forth spraying or a rotary spraying. The spraying device (112) can usually be applied in agricultural irrigation or hygiene control in animal husbandry.

As an implementation of the present embodiment, the device for producing atomizing ozonated water by high pressure (100) also comprises a first valve (114) and a third pipeline (116). In particular, the inlet of the third pipeline (116) is connected with the first pressurizing device (102), and the outlet of the third pipeline (116) is connected with the ozone generator (104). Understandably, the first pressurizing device (102) feeds compressed air into the ozone generator (104) through the third pipeline (116). Furthermore, the first valve (114) is configured on the third pipeline (116). The first valve (114) is used to adjust the pressure of the first pressurizing device (102) on the ozone or the unconverted air in the ozone generator (104), thereby controlling the concentration of atomized ozonated water formed at the spraying device (112).

Specifically, when the amount of water or ozonated water from the water supply device (106) is fixed, the output amount of compressed air from the first pressurizing device (102) can be controlled by the first valve (114), thereby controlling the amount of compressed ozone or unconverted compressed air to be fed into the spraying device (112). Understandably, a varying amount of compressed ozone and a fixed amount of the water can produce atomized ozonated water of varying concentration, and a varying amount of unconverted compressed air and a fixed amount of ozonated water can also produce atomized ozonated water of varying concentration, thereby controlling the concentration of atomized ozonated water formed at the spraying device (112) by the first valve (114).

As an implementation of the present embodiment, the device for producing atomizing ozonated water by high pressure (100) also comprises a second valve (118). In particular, the second valve (118) is configured on second pipeline (110). The second valve (118) is used to adjust the flow rate of the water or ozonated water from the water supply device (106) in order to control the concentration of the atomized ozonated water formed at the spraying device (112).

Specifically, when the amount of compressed ozone or unconverted compressed air from the ozone generator (104) is fixed, the flow rate of water or ozonated water from the water supply device (106) can be controlled by the second valve (118). Understandably, a varying amount of water and a fixed amount of compressed ozone can produce atomized ozonated water of varying concentration, and a varying amount of ozonated water and a fixed amount of unconverted compressed air can also produce atomized ozonated water of varying concentration, thereby controlling the concentration of atomized ozonated water formed at the spraying device (112) by the second valve (118).

Preferably, when the device for producing atomizing ozonated water by high pressure (100) comprises a first valve (114) and a second valve (118), the first valve (114) controls the output amount of compressed ozone or unconverted compressed air from the ozone generator (104) as a variable, and the second valve (118) controls the output amount of the water or ozonated water from the water supply device (106) as a variable, thereby effectively controlling the concentration of atomized ozonated water formed at the spraying device (112).

Please refer to FIG. 2. As an approach, the device for producing atomizing ozonated water by high pressure (100) also comprises a second pressurizing device (120). The second pressurizing device (120) is connected with the water supply device (106). The second pressurizing device (120) is used to pressurize the water supply device (106) to control the flow rate of the water or ozonated water from the water supply device (106), thereby controlling the concentration of the atomized ozonated water formed at the spraying device (112).

Furthermore, the device for producing atomizing ozonated water by high pressure (100) also comprises an ultraviolet lamp. The ultraviolet lamp is configured on the outside of the spraying device (112) to produce ultraviolet light, which reacts with atomized ozonated water to produce hydroxyl radicals for decomposition of toxic substances. Understandably, when atomized ozonated water reacts with ultraviolet light, hydroxyl radicals are produced, which can effectively decompose the VOC and other toxic substances in the air, such as, pesticide or dioxin from incineration, thus achieving the result of air purification.

Furthermore, the device for producing atomizing ozonated water by high pressure (100) also comprises a cooling device. The cooling device is configured inside the water supply device (106). The cooling device is used to cool down the water or ozonated water. Understandably, cooling the water to be used for atomization (to below 15 degrees Celsius) before atomization is more favorable for dissolution of ozone in water. Besides, low temperature ozone water mist produced from low temperature water can effectively and directly cool down the air.

Please refer to FIG. 3. As an implementation of the present embodiment, the device for producing atomizing ozonated water by high pressure (100) also comprises an oxygen generation device (122). In particular, the oxygen generation device (122) is configured between and connected with the first pressurizing device (102) and the ozone generator (104). The oxygen generation device (122) is used to receive compressed air from the first pressurizing device (102), and then feed the oxygen produced from the compressed air into the ozone generator (104) for further treatment. Understandably, no other impurities will be produced when oxygen is converted into ozone in the ozone generator (104), thereby ensuring the purity of the atomized ozonated water mixed and formed at the spraying device (112).

By using the first pressurizing device (102) to feed compressed air into the ozone generator (104), using the ozone generator (104) to receive compressed air and converting part of the compressed air into compressed ozone and feed the compressed ozone or unconverted compressed air into the spraying device (112) through the first pipeline (108), and by using the water supply device (106) to feed water or ozonated water into the spraying device (112) through the second pipeline (110) such that either the water is mixed with the compressed ozone in the spraying device (112) to form atomized ozonated water, or the ozonated water is mixed with unconverted compressed air in the spraying device (112) to form atomized ozonated water, the device for producing atomized ozonated water by high pressure (100) provided by the first embodiment of the present invention provides a cleansing element for air purification, dissolution of chemical gases and assurance of environmental hygiene.

### Second embodiment

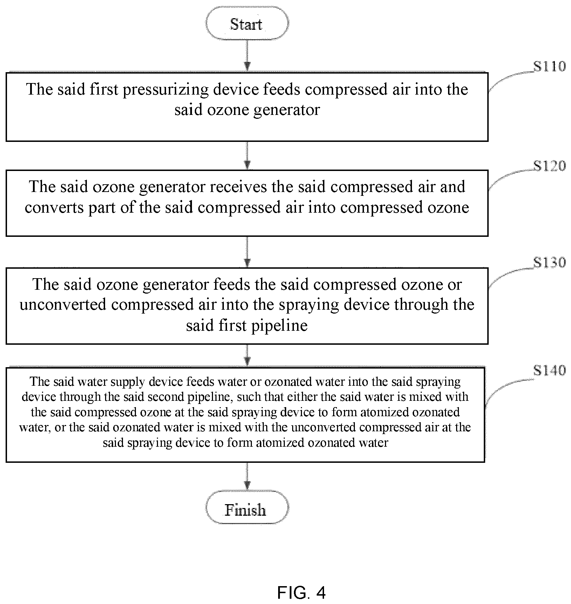

Please refer to FIG. 4. FIG. 4 is a schematic flow chart of a method for producing atomized ozonated water by high pressure provided by the second embodiment of the present invention. The process shown in FIG. 4 is explained below. The method is used in the device for producing atomized ozonated water by high pressure (100). The device for producing atomized ozonated water by high pressure (100) comprises a first pressurizing device (102), an ozone generator (104), a water supply device (106), a first pipeline (108), a second pipeline (110), and a spraying device (112). The first pressurizing device (102) is connected with the ozone generator (104). The inlet of the first pipeline (108) is connected with the ozone generator (104). The outlet of the first pipeline (108) is connected with the spraying device (112). The inlet of the second pipeline (110) is connected with the water supply device (106). The outlet of the second pipeline (110) is connected with the spraying device (112). The said method comprises:
Step S110: The said first pressurizing device feeding compressed air into the said ozone generator.
Step S120: The said ozone generator receiving the said compressed air, converting part of the compressed air into compressed ozone.
Step S130: The said ozone generator feeding the said compressed ozone or unconverted compressed air into the said spraying device through the said first pipeline.
Step S140: The said water supply device feeding water or ozonated water into the said spraying device through the said second pipeline, such that either the said water is mixed with the said compressed ozone in the said spraying device to form atomized ozonated water, or the said ozonated water is mixed with the unconverted compressed air in the spraying device to form atomized ozonated water.

In summary, the device and the method for producing atomized ozonated water by high pressure provided by the embodiments of the present invention comprise a first pressurizing device, an ozone generator, a water supply device, a first pipeline, a second pipeline, and a spraying device. The first pressurizing device is connected with the ozone generator. The inlet of the first pipeline is connected with the ozone generator. The outlet of the first pipeline is connected with the spraying device. The inlet of the second pipeline is connected with the water supply device. The outlet of the second pipeline is connected with the spraying device. By using the first pressurizing device to feed compressed air into the ozone generator, using the ozone generator to receive the compressed air and convert part of the compressed air into compressed ozone and feed the compressed ozone or unconverted compressed air into the spraying device through the first pipeline, and using the water supply device to feed water or ozonated water into the spraying device through the second pipeline, such that either water is mixed with compressed ozone in the spraying device to form atomized ozonated water, or ozonated water is mixed with unconverted compressed air in the spraying device to form atomized ozonated water, a cleansing element is provided for air purification, dissolution of chemical gases and assurance of environmental hygiene.

The above embodiments are only some preferred embodiments of the present invention but are not intended to limit the present invention. For persons skilled in the art, there may be various modifications and changes to the present invention. Any modification, equivalent replacement or improvement within the spirits and principles of the present invention should be included within the scope of protection of the present invention.

## Claims

1. A device for producing atomized ozonated water by high pressure, which is **characterized by** comprising a first pressurizing device, an ozone generator, a water supply device, a first pipeline, a second pipeline, and a spraying device. The said first pressurizing device is connected with the said ozone generator. The inlet of the said first pipeline is connected with the said ozone generator. The outlet of the said first pipeline is connected with the said spraying device. The inlet of the said second pipeline is connected with the said water supply device. The outlet of the said second pipeline is connected with the spraying device.
The said first pressurizing device is used to feed compressed air into the said ozone generator.
The said ozone generator is used receive the said compressed air, convert part of the said compressed air into compressed ozone, and feed the said compressed ozone or unconverted compressed air into the said spraying device through the said first pipeline.
The said water supply device is used to feed water or ozonated water into the said spraying device through the said second pipeline such that either the said water is mixed with the said compressed ozone in the spraying device to form atomized ozonated water, or the said ozonated water is mixed with the said unconverted compressed air in the spraying device to form atomized ozonated water.

2. The said device for producing atomized ozonated water by high pressure according to Claim 1 is **characterized by** comprising a first valve and a third pipeline. The inlet of the said third pipeline is connected with the said first pressurizing device. The outlet of the said third pipeline is connected with the said ozone generator. The said first valve is configured in the said third pipeline. The said first valve is used to adjust the pressure of the first pressurizing device on the ozone or the unconverted air in the said ozone generator in order to control the concentration of atomized ozonated water formed at the said spraying device.

3. The said device for producing atomized ozonated water by high pressure according to Claim 2 is **characterized by** comprising a second valve. The said second valve is configured in said second pipeline. The said second valve is used to adjust the flow rate of the said water or the said ozonated water from the said water supply device in order to control the concentration of the atomized ozonated water formed at the said spraying device.

4. The said device for producing atomized ozonated water by high pressure according to Claim 1 is **characterized by** comprising a second pressurizing device. The said second pressurizing device is connected with the said water supply device. The said second pressurizing device is used to pressurize the said water supply device in order to control the flow rate of the said water or the said ozonated water from the said water supply device.

5. The said device for producing atomized ozonated water by high pressure according to Claim 1 is **characterized by** comprising an ultraviolet lamp. The said ultraviolet lamp is configured on the outside of the said spraying device to produce ultraviolet light, which reacts with the said atomized ozonated water to produce hydroxyl radicals for decomposition of toxic substances.

6. The said device for producing atomized ozonated water by high pressure according to Claim 1 is **characterized by** comprising a cooling device. The said cooling device is configured inside the said water supply device. The said cooling device is used to cool down the said water or the said ozonated water.

7. The said device for producing atomized ozonated water by high pressure according to Claim 1 is **characterized by** comprising an oxygen generation device. The said generation device is configured between and connected with the said first pressurizing device and the ozone generator. The said oxygen generation device is used to receive compressed air from the said first pressurizing device, and then feeding the oxygen generated from the said compressed air into the said ozone generator.

8. The said device for producing atomized ozonated water by high pressure according to Claim 1 is **characterized in that** the said first pressurizing device is an air compressor.

9. The said device for producing atomized ozonated water by high pressure according to Claim 1 is **characterized in that** the said spraying device is either a fix-mounted spraying device or a mobile pipes-spraying device.

10. A method for producing atomized ozonated water by high pressure, which is **characterized by** being applied in a device for producing atomized ozonated water by high pressure. The said device for producing atomized ozonated water comprises a first pressurizing device, an ozone generator, a water supply device, a first pipeline, a second pipeline, and a spraying device. The said first pressurizing device is connected with the said ozone generator. The inlet of the said first pipeline is connected with the said ozone generator. The outlet of the said first pipeline is connected with the said spraying device. The inlet of the said second pipeline is connected with the said water supply device. The outlet of the said second pipeline is connected with the said spraying device. The said method comprises:
The said first pressurizing device feeding compressed air into the said ozone generator;
The said ozone generator receiving the said compressed air, converting part of the compressed air into compressed ozone;
The said ozone generator feeding the said compressed ozone or unconverted compressed air into the said spraying device through the said first pipeline;
The said water supply device feeding water or ozonated water into the said spraying device through the said second pipeline such that either the said water is with the said compressed ozone in the spraying device to form atomized ozonated water, or the ozonated water is mixed with the unconverted compressed air in the spraying device to form atomized ozonated water.
